# EUROPEAN PATENT SPECIFICATION

(11) **EP 2 407 159 B1**
(45) Date of publication and mention of the grant of the patent: **30.10.2013**
(21) Application number: 11174277.1
(22) Date of filing: 15.07.2011
(51) Int. Cl.: A61K 9/48, A61K 31/4178

(54) **Pharmaceutical compositions of olmesartan**
Pharmazeutische Zusammensetzungen aus Olmesartan
Compositions pharmaceutiques d'olmésartan

(30) Priority: 16.07.2010 TR 201005867
(43) Date of publication of application: 18.01.2012
(73) Proprietor: Sanovel Ilaç Sanayi Ve Ticaret Anonim Sirketi, 34460 Sariyer/Istanbul (TR)
(72) Inventor: CIFTER, ÜMIT, 34398 Istanbul (TR); TÜRKYILMAZ, Ali, 34398 Istanbul (TR); TURP, Hasan Ali, 34398 Istanbul (TR); KARAKÖY, Basak Acar, 34398 Istanbul (TR)
(74) Representative: Sevinç, Erkan

(56) References cited:
- WO-A2-2009/125981
- KR-A- 20090 107 958
- DATABASE WPI Week 201057 Thomson Scientific, London, GB; AN 2010-K02426 XP002619426, -& CN 101 766 609 A (BEIJING HOPE HUGE PHARM SCI & TECHNOLOGY) 7 July 2010 (2010-07-07)

## Description

### Technical Aspect

This invention is related to a novel capsule formulation of olmesartan medoxomil as defined by the claims, having an improved manufacturing process and storage stability, furthermore the invention is directed to its use in the treatment of hypertension.

### Background of the Invention

The active ingredient olmesartan medoxomil is a prodrug which is hydrolized to olmesartan during absorbtion from the gastrointestinal tract. Olmesartan is selective AT₁ subtype angiotensin II receptor antagonist.

Olmesartan medoxomil has a chemical name as 2,3-dihydroxy-2-butenyl 4-(1-hydroxy-1-methylethyl)-2-propyl-1-[p-(o-1H-tetrazol-5-ylphenyl)benzyl]imidazole-5-carboxylate, cyclic 2,3-carbonate and its chemical structure is shown in the Formula I.

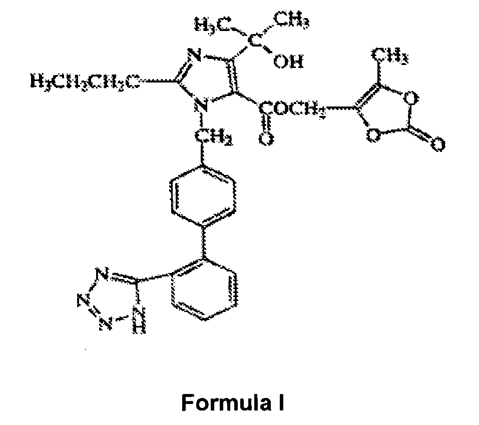

Olmesartan medoxomil is known from the US Patent No. 5,616,599 and is available in the market as film-coated tablet formulations. It is administered orally in a therapeutic dose of 5 mg, 20 mg or 40 mg and is indicated for the treatment of hypertension. It may be used alone or in combination with other antihypertensive agents.

WO 2009/125981-A2 discloses, in general, pharmaceutical formulations consisting of immediate and delayed release compartments for reducing the concerns about side effects, improving drug effects and simplifying the instructions for use of the drug. In one embodiment, olmesartan is provided in an immediate release compartment along with other excipients such as microcrystalline cellulose, lactose, polyvinylpyrrolidone and magnesium stearate in preparation of pharmaceutical capsules.

CN 101 766 609-A and KR 20090107958-A describe formulations in tablet or capsule forms, comprising olmesartan and amlodipine for combined treatment along with other excipients such as croscarmellose sodium, magnesium stearate and microcrystalline cellulose. None of the above referenced documents however provide a complete formulation for attaining an olmesartan capsule formulation having better flow and uniformity characteristics.

Many tablet formulations of olmesartan medoxomil and processes thereof is known from the prior art. In PCT application WO 2007/128478 A2, oral tablet compositions of olmesartan medoxomil are disclosed. This application is directed to stable tablet formulations in the pH range 3-5 but there is nothing disclosed about capsule formulations of olmesartan medoxomil or its process therof.

It is known that, it is difficult to formulate olmesartan medoxomil as oral solid dosage formulations because of its physico chemical properties. In tablet formulations of olmesartan a compressing force or compactor is used to have a compact powder mixture to obtain tablet formulations. But this may cause capping in finished dosage tablet forms or a decrease can be observed in dissolution rate especially during the shelf life. Another important problem is its degredation, because under high pressure and heat it can easily convert to its degredation products.

Therefore, there is a need in the art for solid oral dosage formulations of olmesartan medoxomil having an improved dissolution rate with improved processes and which are more stable during the shelf life. Accoring to the present invention novel capsule formulations of olmesartan medoxomil with improved stability and process are provided.

Further advantages and embodiments of the present invention will become apparent from the following description.

### Detailed Description of the Invention

The present invention provides a novel capsule formulation of olmesartan medoxomil as defined by the claims which overcomes the above described problems in prior art and have additive advantages over them.

The main object of the present invention is to provide stable capsule formulations of olmesartan medoxomil having high dissolution rate throughout the shelf-life.

Another object is to provide improved manufacturing processes which is simple, cost-effective and time saving for preparing the capsule formulations of olmesartan medoxomil.

A further object is to obtain an adequate uniformity of low dosage forms of olmesartan medoxomil with using adequate excipients and improved processes.

In prior art, pharmaceutical compositions of olmesartan modexomil mostly have low-substituted hydroxypropyl cellulose and lactose with other common known excipients. However these excipients have poor flow characteristics and tend to increase powder density if used in high concentrations. When the powder density increase it is diffucult to direct press as a tablet or to fill in a capsule and a compaction force is needed which may cause unstable and having poor bioavailability tablet dosage formulations of olmesartan medoxomil.

Surprisingly, it is found that when olmesartan medoxomil capsule formulation, is prepared with polyvinyl pyrrolidone and crosscarmellose sodium in a specific weight ratio the powder density is not increased and there is no need to compact the powder by using a compactor to fill in a capsule. Therefore, it is important not to use a compactor during the manufacturing process because of olmesartan's degredation products. It can easily affected by the pressure and the temperature and it can be easily tend to degredate during the shelf-life which may cause toxicity.

Additionally, the improved flow and lubricity characteristics of both polyviniylpyrrolidone and crosscarmellose sodium can impart further benefit to the formulation in a very cost-effective manner.

In this regard, the weight ratio of the polyvinylpyrrolidone and croscarmellose sodium is between 1:10 and 10:1 (w/w). Preferably the weight ratio is between 1:5 and 5:1 (w/w) and more preferably the weight ratio is 3:5 (w/w).

In one embodiment, olmesartan medoxomil is present in an amount of 0.1 to 15.0% by weight of total capsule composition; preferably it is 5.0 to 10.0% by weight of total composition.

Another object of this present invention is a capsule formulation of olmesartan medoxomil comprising microcrystalline cellulose. It is objected to use microcrystalline cellulose with olmesartan medoxomil in a specific weight ratio to overcome the technical problems which are shown above. According to this object, when the weight ratio of olmesartan medoxomil to microcrystalline cellulose is in between 1:20 to 20:1 (w/w) the capsule formulation has better storage stability. Preferably the weight ratio is 1:10 to 10:1 (w/w). Therefore, good storage properties obtained without using any other preservative agents which is very important to have during the shelf-life because of olmesartan's degredation products.

According to another preferred embodiment of the present invention, a synergistic effect is observed over the dissolution profile and homogeneity of the doses of olmesartan medoxomil throughout other pharmaceutical excipients. Therefore, magnesium stearate and colloidal silicon dioxide is used in a specific weight ratio wherein the weight ratio is between 1:10 to 15:1 (w/w), preferably it is 1:1 to 10:1 (w/w), more preferably it is 5:1 to 8:1 (w/w). Thus, a proper dosage and homogeneity is ensured and the final dosage forms have high and adequate content uniformity with improved bioavailability.

In one embodiment, the capsule formulations of olmesartan medoxomil comprise at least one pharmaceutically acceptable excipient. Suitable pharmaceutically acceptable excipients comprise but are not limited to fillers & diluents, lubricants and glidants.

Suitable fillers and/or diluents may comprise but not limited to cellulose derivatives (e.g. powdered cellulose, microcrystalline cellulose), starch derivatives (e.g. corn starch, pregelatinized starch), calcium carbonate, magnesium carbonate, sucrose, fructose, lactose, polysaccharides (e.g. dextrose), xylitol, mannitol and the like and mixtures thereof. The most preffered fillers and/or diluents are cellulose derivatives such as microcrystalline cellulose.

Suitable lubricants may comprise but not limited to magnesium stearate, sodium stearyl fumarate, polyethylene glycol, stearic acid, metal stearates, talc, boric acid, sodium chloride benzoate and acetate, sodium or magnesium lauryl sulfate and the like and mixtures thereof, preferably the lubricant is magnesium stearate.

Suitable glidants may comprise but not limited to colloidal silicon dioxide, talc, aluminium silicate and the like and mixtures thereof, preferably the glidant is colloidal silicon dioxide.

In one aspect, the pharmaceutical composition of olmesartan medoxomil comprises,
a. 0.1 - 15.0 % of olmesartan medoxomil
b. 5.0 - 90 % of microcrystalline cellulose
c. 0.1 - 25 % of polyvinylpyrolidone
d. 0.1 - 15.0 % croscarmellose sodium
e. 0.01 - 10.0 % of magnesium stearate
f. 0.01 - 5.0 % of colloidal silicon dioxide

In one of the preffered embodiments of this invention, the final dosage form of the capsule formulation of olmesartan medoxomil is tested by uniformity dosage unit method of Ph. Eur 2.9.40. According to this method, minimum 30 dosage unit are tested, and 10 dosage unit is taken from these tested dosage unit and amount of active agent is analysed by the proper referenced method and calculated. Acceptance crieteria should be less than 15 to prove that the capsules are filled uniformly. Suprisingly, it is found that, the final dosage forms of the capsule formulation of this invention has an acceptance criteria of less than 15 according to the uniformity of dosage units (Ph. Eur 2.9.40)

In one of the preffered embodiments of this invention the pharmaceutical composition of olmesartan medoxomil may have a long-term shelf-life of 24 months or more at ambient temperature, in its original packaging.

In one embodiment, the pharmaceutical compositions according to the present invention may further comprise one or more other antihypertensive agent such as a thiazide diuretic.

In one embodiment, the capsule formulations of olmesartan medoxomil are tested by their dissolution profiles, in phosphate buffer at pH 6.8 and 37°C using a USP paddle method rotating at 50 RPM and 70 % or more of the olmesartan medoxomil is dissolved in 30 min.

In one embodiment, the process for preparing the capsule formulation of olmesartan medoxomil, comprising the following steps;
a. blending olmesartan medoxomil with microcrystalline cellulose, polyvinylpyrrolidone, croscarmellose sodium and colloidal silicon dioxide,
b. sieving this powder mixture
c. adding magnesium stearate to this mixture and final blending
d. filling the powder mixture into capsules.

This process is important because it does not comprise the step of pressing the powder mixture by a compactor which is very important because of the stability of the capsule formulations. Because, olmesartan medoxomil is difficult to formulate because it is very sensitive to heat and pressure and it may easily degrade to its impurities.

This invention is further defined by reference to the following example. Although the example is not intended to limit the scope of the present invention, they should be considered in the light of the description detailed above.

### Example 1 : Capsule formulation of olmesartan medoxomil

| **Ingredients** | **Amount %** |
|---|---|
| Olmesartan medoxomil | 9.52 |
| Microcrystalline cellulose (Avicel pH-112) | 80.98 |
| Polyvinylpyrollidone 25 | 3.00 |
| Croscarmellose sodium | 5.00 |
| Magnesium stearate | 1.30 |
| Colloidal silicon dioxide | 0.20 |

The formulation of this example is manufactured according to the process described detailed above in the description.

### Example 2 : Dissolution Profile

The pharmaceutical composition of this present invention (Example 1, 12 capsules) was tested by its dissolution profile in phosphate buffer at pH 6.8 and 37°C using a USP paddle method rotating at 50 RPM and 70 % or more of the olmesartan medoxomil is dissolved in 30 min. The results are shown below in Table 1.

**Table 1**

| **Time (min.)** | **Olmesartan medoxomil (%)** |
|---|---|
| 5 | 55,2 |
| 10 | 68,4 |
| 15 | 73,0 |
| 20 | 75,5 |
| 30 | 77,9 |
| 45 | 79,8 |
| 60 | 78,4 |

### Example 3 : Uniformity of dosage units according to Ph. Eur 2.9.40

The pharmaceutical composition of this present invention (Example 1) was tested by uniformity dosage unit method of Ph. Eur 2.9.40. According to this method, minimum 30 dosage unit are tested, and 10 dosage unit is taken from these tested dosage unit and amount of active agent is analysed by the proper referenced method and calculated. Acceptance crieter should be less than 15 to prove that the capsules are filled uniformly. The results are shown below in Table 1.

**Table 2**

| **Sample No** | **Uniformity of dosage units Olmesartan Medoxomil content (%)** |
|---|---|
| 1 | 98.40 |
| 2 | 98.20 |
| 3 | 97.50 |
| 4 | 96.40 |
| 5 | 98.10 |
| 6 | 98.60 |
| 7 | 97.80 |
| 8 | 96.50 |
| 9 | 96.40 |
| 10 | 94.50 |
| **mean** | **97.2** |
| **RSD %** | **1.31** |
| **Acceptance criteria (<15)** | **4.4** |

### Example 4 : Disintegration Test

The pharmaceutical composition of this present invention (Example 1) was tested by its disintegration. The results are shown below in Table 3.

**Table 3**

| **Sample No** | **Disintegration time** |
|---|---|
| 1 | < 2 min. |
| 2 | < 2 min. |
| 3 | < 2 min. |
| 4 | < 2 min. |
| 5 | < 2 min. |
| 6 | < 2 min. |
| 7 | < 2 min. |
| 8 | < 2 min. |
| 9 | < 2 min. |
| 10 | < 2 min. |

## Claims

1. A capsule formulation of olmesartan medoxomil, comprising polyvinylpyrrolidone and croscarmellose sodium in a weight ratio between 1:10 to 10:1 (w/w); and magnesium stearate and colloidal silicon dioxide in a weight ratio between 1:10 to 15:1 (w/w).

2. The capsule formulation of olmesartan medoxomil according to the claim 1, wherein the weight ratio of polyvinylpyrrolidone and croscarmellose sodium is 1:5 to 5:1 (w/w).

3. The capsule formulation of olmesartan medoxomil according to the claim 2, wherein the weight ratio of polyvinylpyrrolidone and croscarmellose sodium is 3:5 (w/w).

4. The capsule formulation of olmesartan medoxomil according to the claims 1 to 3, wherein olmesartan medoxomil is present in an amount of 0.1 to 15.0 % by weight of total composition; preferably it is 5.0 to 10.0 % by weight of total composition.

5. The capsule formulation of olmesartan medoxomil according to the claims 1 to 4, comprising microcrystalline cellulose wherein the weight ratio of olmesartan medoxomil to microcrystalline cellulose is 1:20 to 20:1 (w/w), preferably 1:10 to 10:1 (w/w).

6. The capsule formulation of olmesartan medoxomil according to the claims 1 to 5, further comprising at least one pharmaceutically acceptable excipient wherein the excipient is selected from the group comprising fillers & diluents, lubricants and glidants.

7. The capsule formulation of olmesartan medoxomil according to the claim 6, wherein the filler and/or diluent is selected from the group comprising cellulose derivatives (e.g. powdered cellulose, microcrystalline cellulose), starch derivatives (e.g. corn starch, pregelatinized starch), calcium carbonate, magnesium carbonate, sucrose, fructose, lactose, polysaccharides (e.g. dextrose), xylitol, mannitol and the like and mixtures thereof, preferably the filler and/or diluent is a cellulose derivative such as microcrystalline cellulose.

8. The capsule formulation of olmesartan medoxomil according to the claim 6, wherein the lubricant is selected from the group comprising magnesium stearate, sodium stearyl fumarate, polyethylene glycol, stearic acid, metal stearates, talc, boric acid, sodium chloride benzoate and acetate, sodium or magnesium lauryl sulfate and the like and mixtures thereof; preferably the lubricant is magnesium stearate.

9. The capsule formulation of olmesartan medoxomil according to the claim 6, wherein the glidant is selected from the group comprising colloidal silicon dioxide, talc, aluminium silicate and the like and mixtures thereof; preferably the glidant is colloidal silicon dioxide.

10. The capsule formulation of olmesartan medoxomil according to the claims 8 and 9, wherein the weight ratio of magnesium stearate and colloidal silicon dioxide is between 1:1 to 10:1 (w/w), preferably it is between 5:1 to 8:1 (w/w).

11. The capsule formulation of olmesartan medoxomil according to any preceding claims, comprising;
a. 0.1 -15.0 % of olmesartan medoxomil
b. 5.0 - 90 % of microcrystalline cellulose
c. 0.1 - 25 % of polyvinylpyrolidone
d. 0.1 -15.0 % croscarmellose sodium
e. 0.01 - 10.0 % of magnesium stearate
f. 0.01 - 5.0 % of colloidal silicon dioxide

12. The capsule formulation of olmesartan medoxomil according to any preceding claims, wherein the pharmaceutical composition has a long term shelf-life of 24 months or more at ambient temperature, in its original packaging.

13. The capsule formulation of olmesartan medoxomil according to any preceding claims, wherein 70 % or more of the olmesartan medoxomil is dissolved in 30 min in phosphate buffer at pH 6.8, at 37°C using a USP paddle method rotating at 50 RPM.

14. A process for preparing the capsule formulation of olmesartan medoxomil according to any preceding claims, comprising the following steps;
g. blending olmesartan medoxomil with microcrystalline cellulose, polyvinylpyrrolidone, croscarmellose sodium and colloidal silicon dioxide,
h. sieving this powder mixture
i. adding magnesium stearate to this mixture and final blending
j. filling the powder mixture into capsules.

15. The process according to claim 14, further not comprising the step of pressing the powder mixture by a compactor.

## Patentansprüche

1. Kapselformulierung von Olmesartanmedoxomil, umfassend Polyvinylpyrrolidon und Croscarmellose-Natrium in einem Gewichtsverhältnis zwischen 1:10 bis 10:1 (w/w); und Magnesiumstearat und kolloidales Siliziumdioxid in einem Gewichtsverhältnis zwischen 1:10 bis 15:1 (w/w).

2. Kapselformulierung von Olmesartanmedoxomil nach Anspruch 1, wobei das Gewichtsverhältnis von Polyvinylpyrrolidon und Croscarmellose-Natrium 1:5 bis 5:1 (w/w) beträgt.

3. Kapselformulierung von Olmesartanmedoxomil nach Anspruch 2, wobei das Gewichtsverhältnis von Polyvinylpyrrolidon und Croscarmellose-Natrium 3:5 (w/w) beträgt.

4. Kapselformulierung von Olmesartanmedoxomil nach Ansprüchen 1 bis 3, wobei Olmesartanmedoxomil in einer Menge von 0,1 bis 15,0 Gewichtsprozent der gesamten Zusammensetzung vorliegt, vorzugsweise beträgt sie 5,0 bis 10,0 Gewichtsprozent der gesamten Zusammensetzung.

5. Kapselformulierung von Olmesartanmedoxomil nach Ansprüchen 1 bis 4, umfassend mikrokristalline Cellulose, wobei das Gewichtsverhältnis von Olmesartanmedoxomil zu mikrokristalliner Cellulose 1:20 bis 20:1 (w/w), vorzugsweise 1:10 bis 10:1 (w/w) beträgt.

6. Kapselformulierung von Olmesartanmedoxomil nach Ansprüchen 1 bis 5, des Weiteren umfassend mindestens einen pharmazeutisch verträglichen Hilfsstoff, wobei der Hilfsstoff ausgewählt ist aus der Gruppe umfassend Füllstoffe und Verdünnungsmittel, Schmiermittel und Fließregulierungsmittel.

7. Kapselformulierung von Olmesartanmedoxomil nach Anspruch 6, wobei der Füllstoff und/oder das Verdünnungsmittel ausgewählt ist aus der Gruppe umfassend Cellulosederivate (z.B. pulverisierte Cellulose, mikrokristalline Cellulose), Stärkederivate (z.B. Maisstärke, vorgelierte Stärke), Calciumcarbonat, Magnesiumcarbonat, Saccharose, Fruktose, Laktose, Polysaccharide (z.B. Dextrose), Xylitol, Mannitol und dergleichen und Mischungen davon, vorzugsweise ist der Füllstoff und/oder das Verdünnungsmittel ein Cellulosederivat wie mikrokristalline Cellulose.

8. Kapselformulierung von Olmesartanmedoxomil nach Anspruch 6, wobei das Schmiermittel ausgewählt ist aus der Gruppe umfassend Magnesiumstearat, Natriumstearylfumerat, Polyethylenglycol, Stearinsäure, Metallstearate, Talk, Borsäure, Natriumchloridbenzoat und Acetat, Natrium- oder Magnesiumlaurylsulfat und dergleichen und Mischungen davon; vorzugsweise ist das Schmiermittel Magnesiumstearat.

9. Kapselformulierung von Olmesartanmedoxomil nach Anspruch 6, wobei das Fließregulierungsmittel ausgewählt ist aus der Gruppe umfassend kolloidales Siliziumdioxid, Talk, Aluminiumsilikat und dergleichen und Mischungen davon; vorzugsweise ist das Fließregulierungsmittel kolloidales Siliziumdioxid.

10. Kapselformulierung von Olmesartanmedoxomil nach Ansprüchen 8 und 9, wobei das Gewichtsverhältnis von Magnesiumstearat und kolloidalem Siliziumdioxid zwischen 1:1 bis 10:1 (w/w) liegt, vorzugsweise liegt es zwischen 5:1 bis 8:1 (w/w).

11. Kapselformulierung von Olmesartanmedoxomil nach einem beliebigen der vorhergehenden Ansprüche, umfassend;
a. 0,1 - 15,0 % von Olmesartanmedoxomil
b. 5.0 - 90 % von mikrokristalliner Cellulose
c. 0,1 - 25 % von Polyvinylpyrrolidon
d. 0,1 - 15,0 % Croscarmellose-Natrium
e. 0,01 - 10,0 % Magnesiumstearat
f. 0,01 - 5,0 % von kolloidalem Siliziumdioxid.

12. Kapselformulierung von Olmesartanmedoxomil nach einem beliebigen der vorhergehenden Ansprüche, wobei die pharmazeutische Zusammensetzung eine Langzeit-Lagerfähigkeit von 24 Monaten oder mehr bei Umgebungstemperatur in seiner originalen Verpackung aufweist.

13. Kapselformulierung von Olmesartanmedoxomil nach einem beliebigen der vorhergehenden Ansprüche, wobei 70% oder mehr des Olmesartanmedoxomils in 30 Min. in Sulfatpuffer bei pH 6,8 bei 37°C unter Verwendung eines USB Paddle-Verfahrens bei 50 RPM rotierend aufgelöst wird.

14. Verfahren zur Herstellung der Kapselformulierung von Olmesartanmedoxomil nach einem beliebigen der vorhergehenden Ansprüche, umfassend die folgenden Schritte;
g. Vermischen von Olmesartanmedoxomil mit mikrokristalliner Cellulose, Polyvinylpyrrolidon, Croscarmellose-Natrium und kolloidalem Siliziumdi- oxid,
h. Sieben dieser Pulvermischung
i. Hinzufügen von Magnesiumstearat zu dieser Mischung und abschließendes Vermischen
j. Füllen der Pulvermischung in Kapseln.

15. Verfahren nach Anspruch 14, des Weiteren nicht umfassend den Schritt von Pressen der Pulvermischung durch einen Kompaktor.

## Revendications

1. Formulation de capsule d'olmésartan médoxomil, comprenant de la polyvinylpyrrolidone et de la croscarmellose sodique dans un rapport pondéral compris entre 1:10 et 10:1 (poids/poids) ; et du stéarate de magnésium et du dioxyde de silicium colloïdal dans un rapport pondéral compris entre 1:10 et 15:1 (poids/poids).

2. Formulation de capsule d'olmésartan médoxomil selon la revendication 1, dans laquelle le rapport pondéral de la polyvinylpyrrolidone et de la croscarmellose sodique est de 1:5 à 5:1 (poids/poids).

3. Formulation de capsule d'olmésartan médoxomil selon la revendication 2, dans laquelle le rapport pondéral de la polyvinylpyrrolidone et de la croscarmellose sodique est de 3:5 (poids/poids).

4. Formulation de capsule d'olmésartan médoxomil selon les revendications 1 à 3, dans laquelle l'olmésartan médoxomil est présent en une quantité de 0,1 à 15,0 % du poids total de la composition ; de préférence, il représente de 5,0 à 10,0 % du poids total de la composition.

5. Formulation de capsule d'olmésartan médoxomil selon les revendications 1 à 4, comprenant de la cellulose microcristalline dans laquelle le rapport pondéral d'olmésartan médoxomil et de la cellulose microcristalline est de 1:20 à 20:1 (poids/poids), de préférence de 1:10 à 10:1 (poids/poids).

6. Formulation de capsule d'olmésartan médoxomil selon les revendications 1 à 5, comprenant en outre au moins un excipient pharmaceutiquement compatible, dans laquelle l'excipient est sélectionné parmi le groupe comprenant des diluants et charges, des lubrifiants et des agents de glissement.

7. Formulation de capsule d'olmésartan médoxomil selon la revendication 6, dans laquelle le diluant et/ou charge est sélectionné parmi le groupe comprenant des dérivés de cellulose (par exemple, la cellulose en poudre, la cellulose microcristalline), des dérivés d'amidon (par exemple, l'amidon de maïs, l'amidon pré-gélatinisé), le carbonate de calcium, le carbonate de magnésium, le sucrose, le fructose, le lactose, les polysaccharides (par exemple, le dextrose), le xylitol, le mannitol et analogues et leurs mélanges, de préférence, le diluant et/ou charge est un dérivé de cellulose tel que la cellulose microcristalline.

8. Formulation de capsule d'olmésartan médoxomil selon la revendication 6, dans laquelle le lubrifiant est sélectionné parmi le groupe comprenant le stéarate de magnésium, le fumarate stéarylique de sodium, le polyéthylène glycol, l'acide stéarique, les stéarates métalliques, le talc, l'acide borique, les chlorure, benzoate et acétate de sodium, le sulfate laurique de sodium ou de magnésium et analogue ainsi que leurs mélanges ; de préférence, le lubrifiant est du stéarate de magnésium.

9. Formulation de capsule d'olmésartan médoxomil selon la revendication 6, dans laquelle l'agent de glissement est sélectionné parmi le groupe comprenant le dioxyde de silicium colloïdal, le talc, le silicate d'aluminium et analogues ainsi que leurs mélanges ; de préférence, l'agent de glissement est du dioxyde de silicium colloïdal.

10. Formulation de capsule d'olmésartan médoxomil selon les revendications 8 et 9, dans laquelle le rapport pondéral du stéarate de magnésium et du dioxyde de silicium colloïdal est compris entre 1:1 et 10:1 (poids/poids), de préférence, il est compris entre 5:1 et 8:1 (poids/poids).

11. Formulation de capsule d'olmésartan médoxomil selon l'une quelconque des revendications précédentes, comprenant :
a. de 0,1 à 15,0 % d'olmésartan médoxomil
b. de 5,0 à 90 % de cellulose microcristalline
c. de 0,1 à 25 % de polyvinylpyrolidone
d. de 0,1 à 15,0 % de croscarmellose sodique
e. de 0,01 à 10,0 % de stéarate de magnésium
f. de 0,01 à 5,0 % de dioxyde de silicium colloïdal

12. Formulation de capsule d'olmésartan médoxomil selon l'une quelconque des revendications précédentes, dans laquelle la composition pharmaceutique présente une durée de conservation à long terme supérieure ou égale à 24 mois, à température ambiante, dans son conditionnement d'origine.

13. Formulation de capsule d'olmésartan médoxomil selon l'une quelconque des revendications précédentes, dans laquelle 70 % ou plus de l'olmésartan médoxomil sont dissous en 30 mn dans un tampon de phosphate à un pH de 6,8, à 37°C en utilisant un procédé USP par palette tournant à 50 t/mn.

14. Procédé de préparation de la Formulation de capsule d'olmésartan médoxomil selon l'une quelconque des revendications précédentes, comprenant les étapes suivantes :
g. mélange d'olmésartan médoxomil avec de la cellulose microcristalline, de la polyvinylpyrrolidone, de la croscarmellose sodique et du dioxyde de silicium colloïdal,
h. tamisage de ce composé pulvérulent,
i. ajout de stéarate de magnésium à ce composé et mélange final,
j. remplissage des capsules avec le mélange pulvérulent.

15. Procédé selon la revendication 14, ne comprenant pas en outre l'étape de pressage du mélange pulvérulent par un compacteur.
